(19) **Europäisches Patentamt**
**European Patent Office**
**Office européen des brevets**

(11) **EP 2 646 005 B1**

(12) **EUROPEAN PATENT SPECIFICATION**

(45) Date of publication and mention
of the grant of the patent:
**26.06.2019 Bulletin 2019/26**

(21) Application number: **11822806.3**

(22) Date of filing: **28.11.2011**

(51) Int Cl.:
**A61K 9/20** *(2006.01)*    **A61K 9/14** *(2006.01)*

(86) International application number:
**PCT/EP2011/071186**

(87) International publication number:
**WO 2012/072580 (07.06.2012 Gazette 2012/23)**

(54) **COMPRESSED FORMULATIONS OF ORDERED MESOPOROUS SILICAS**

KOMPRIMIERTE FORMULIERUNGEN GEORDNETER MESOPORÖSER SILIKAMATERIALIEN

FORMULATIONS COMPRESSÉES DE SILICES MÉSOPOREUSES ORDONNÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2010 EP 10192938**

(43) Date of publication of application:
**09.10.2013 Bulletin 2013/41**

(73) Proprietor: **Formac Pharmaceuticals N.V.**
**3001 Heverlee (BE)**

(72) Inventors:
• **VIALPANDO, Monica**
**B-3000 Leuven (BE)**
• **MARTENS, Johan**
**B-3040 Huldenberg (BE)**
• **VAN DEN MOOTER, Guy**
**B-3212 Pellenberg (BE)**
• **KIEKENS, Filip**
**B-2440 Geel (BE)**

(74) Representative: **Paemen, Liesbet R.J. et al**
**De Clercq & Partners cvba**
**Edgard Gevaertdreef 10 a**
**9830 Sint-Martens-Latem (BE)**

(56) References cited:
**WO-A2-2009/133100    US-A1- 2006 018 966**
**US-B1- 6 592 764**

• **MONICA VIALPANDO ET AL: "Evaluation of ordered mesoporous silica as a carrier for poorly soluble drugs: Influence of pressure on the structure and drug release", JOURNAL OF PHARMACEUTICAL SCIENCES, vol. 100, no. 8, 1 August 2011 (2011-08-01), pages 3411-3420, XP055022167, ISSN: 0022-3549, DOI: 10.1002/jps.22535**

**Description**

Field of the Invention

This invention concerns compressed formulations comprising ordered mesoporous silica loaded with an active ingredient, a combination of microcrystalline cellulose and croscarmellose sodium, and optional other excipients, wherein said ordered mesoporous silica has been subjected to a calcination step.

Background of the Invention

Ordered mesoporous silica (OMS) materials since long have attracted substantial interest in a broad range of applications such as catalysis, non-linear optics, and molecular adsorption. These materials display an array of uniform mesopores of 2-50 nm in diameter and show good hydrothermal and chemical stability. Mesoporous molecular sieves have regularly arranged channels larger than those of existing zeolites, thus enabling their application in adsorption, isolation, or catalyst conversion reactions of relatively large molecules.

More recently, interest in the development of OMS materials as an oral drug delivery system is rapidly growing. Their large pore volume (up to 2.5 mL/g) in combination with a large specific surface area (up to 1,000 $m^2$/g) results in a high drug-loading capacity. In addition, pore size and surface chemistry can be modified during synthesis to fit the application needs of the user.

Many of the recently developed drugs suffer from poor aqueous solubility, which leads to incomplete dissolution throughout the gastro-intestinal (GI) tract, resulting in low and variable bioavailability. During the mid-1990s, it was reported that roughly 40 % of new drug candidates failed during development due to poor bioavailability, with numbers increasing. Therefore, advancement in innovative approaches to overcome this important formulation challenge is critically needed, making the development of new drug delivery systems highly desirable.

The pore structure of OMS materials is the key attribute to improving the dissolution rate of poorly soluble drugs. Because the pores are only a few times larger than drug molecules, the drug is confined and unable to crystallize. In this form, compounds exhibit higher dissolution rates when compared to their crystalline state, especially when the solubility is limited by high lattice energy. This in turn increases oral bioavailability, as shown by Mellaerts et al. (Eur J Pharm Biopharm 69 : 223-230, 2008).

Because of their ease of manufacture and administration, formulations in the form of tablets still represent the most attractive dosage forms for pharmaceutical and some other types of active ingredients.

Published US application No. 2006/0018966 describes room temperature ionic liquid (RITL) templated porous silicate bodies, having one or more RTIL cations and one or more functionalized organic groups in one or more pores. The average pore diameter of these materials varies between about 1 to about 4 nm. Example 7 illustrates a formulation of such material containing croscarmellose sodium and microcrystalline cellulose.

US 6592764 discloses ordered mesoporous materials prepared using amphiphilic block copolymers, such as triblock polyoxyalkylenes, as templating agents in acidic media followed by calcination at high temperatures. One such material is SBA-15 mesoporous silica material, which has an ordered, two-dimensional hexagonal (p6mm) honeycomb mesostructure.

WO 2009/133100 discloses ordered mesoporous materials prepared under slightly acidic or neutral conditions.

Ghedini et al. (Micropor and Mesopor Mater 132:258-267, 2010) evaluated the response of MCM-41, SBA-15, and silica gel to pressure, but only one pressure value was applied and their assessment was limited to a controlled release formulation.

It now has been found that the pressure required to compress silica materials loaded with an active ingredient, such as for producing a tablet, negatively affects drug release. Drug release from the silica was found to decrease with increasing pressure. Inadequate release from a tablet is undesirable in that larger quantities of active ingredient are required to achieve the same blood plasma levels. Fluctuations in drug release due to variations in the pressure in the compressing step should also be avoided because of the concomitant variations in drug plasma levels.

None of the above mentioned references teaches or suggests the use of a combination of microcrystalline cellulose and croscarmellose sodium in compressed formulations containing active ingredient, loaded ordered mesoporous materials, to improve the release of an active ingredient.

It is an object of the present invention to provide compressed formulations such as tablets, with drug-loaded mesoporous silica materials from which drug release remains unchanged within acceptable boundaries, or even remains completely unchanged, as compared to drug release from non-compressed materials. It is a further object to provide tablet formulations in which drug release is independent within acceptable boundaries, or even remains completely independent, from the pressure exerted in the compression step.

## Summary of the Invention

The present invention is based on the finding that the addition of a combination of microcrystalline cellulose and croscarmellose sodium to the ingredient mixture to be compressed, said mixture comprising active ingredient-loaded silica material and, optionally, other excipients, completely or to an acceptable extend overcomes the problems of reduced release of the active ingredient and the variability in the release in function of compressing pressures.

In one aspect, the present invention concerns a compressed formulation comprising an ordered mesoporous silica loaded with an active ingredient and a combination of microcrystalline cellulose (MCC) and croscarmellose sodium, and optional further excipients, wherein said ordered mesoporous silica has been subjected to a calcination step. The ordered mesoporous silica may have a pore diameter of about 5 to about 30 nm.

In one embodiment the compressed formulation takes the form of a tablet.

In one embodiment, said compressed formulation is for pharmaceutical use, either human or veterinary. In a further embodiment said compressed formulation is a tablet formulation.

In another embodiment, the tablet formulation is for immediate release.

In another embodiment, the ordered mesoporous silica is a hexagonally-ordered two-dimensional array of uniform sized cylindrical pores with a diameter of about 5 to about 30 nm, or a diameter of about 6 to about 16 nm, or a diameter of about 6 to about 14 nm.

In another embodiment, the ordered mesoporous silica has a wall thickness in the range of about 4 - 14 nm, in particular in the range of about 4 - 10 nm, or in the range of about 4 - 8 nm.

In a further aspect, the invention concerns a process for preparing a compressed formulation as specified herein, said process comprising compressing a mixture of anordered mesoporous silica loaded with an active ingredient, a combination of microcrystalline cellulose and croscarmellose sodium, and optional further excipients.

In still a further aspect, it is disclosed a mixture of an ordered mesoporous silica loaded with an active ingredient, a combination of microcrystalline cellulose and croscarmellose sodium, and optional excipients.

## Description of the Figures

Figure 1: Micrographs of COK-12 loaded with itraconazole (ITZ) (a) SEM of non-compressed (b) SEM after 480 MPa compression, (c) TEM of non-compressed, (d) TEM after 480 MPa compression.

Figure 2: SAXS patterns of loaded SBA-15 (top) and COK-12 (bottom) compressed at (□) 0 MPa, (◊) 72 MPa, and (○) 120 MPa.

Figure 3: Nitrogen adsorption/desorption isotherms for COK-12 unloaded (top) and loaded (bottom) samples compressed at (□) 0 MPa, (◊) 72 MPa, and (○) 120 MPa.

Figure 4: DSC thermograms of ITZ and loaded OMS (a) SBA-15, (b) COK-15, (c) SBA-15 after 480 MPa and (d) COK-12 after 480 MPa.

Figure 5: Release profiles of ITZ loaded in OMS and compressed at (●) 0 MPa, (○) 72 MPa, (♦) 120 MPa, (◊) 240 MPa, (■) 360 MPa, and (□) 480 MPa, and (-) crystalline ITZ in SGF + 0.5 % wt. SLS (n = 3, mean ± sd).

Figure 6: Release profiles from tablets containing ITZ loaded in OMS mixed with MCC at concentrations of (■/□) 7/3, (▲/△) 1/1, and (●) 3/7 (top), as well as such to which croscarmellose sodium has been added (Ac-Di-Sol® or AC) at (■/□) 66.5/28.4/5.1, (▲/△) 47.4/47.6/5, and (●/○) 28.5/66.7/4.8 (bottom). Filled and empty symbols refer to 0 MPa and 120 MPa, respectively. SGF + 0.5 % wt. SLS (n = 3, mean ± sd).

Figure 7: Release profiles from tablets containing 20% w/w ezetemibe/COK-12, MCC, and 5% w/w croscarmellose sodium in 900ml 0,01 M pH 7 phosphate buffer + 0,1% SLS

Figure 8: Release profiles from tablets containing 20% w/w ezetemibe/COK-12, MCC, and 10% croscarmellose sodium in 900ml 0,01M pH 7 phosphate buffer + 0,1% SLS.

Figure 9: Release from tablets containing nebivolol/COK12 in 900 ml 0.1N HCl at 50 rpm.

Figure 10: Release from tablets containing tadalafil/COK-12 in 900 ml SGF at 50rpm.

## Detailed Description of the Invention

The term "drug" is meant to be equivalent to the terms "pharmaceutical active ingredient" or "active ingredient". Drugs can be for human or for veterinary use.

As used herein the terms "tablet" or "tablet formulation" refer to any compressed dosage form for administration of an active ingredient to a human or warm-blooded animal. Tablets may be for administration orally, sublingually, rectally, vaginally, or by implantation. They may take any shape or size known in the pharmaceutical art, such as round, oblong, capsule-shaped, or any other known form, and include caplets, minitablets, microtablets, and the like. If desired, the tablet may be covered with a polymer coating.

The terms "mesopore" or "mesoporous" and the like refer to porous structures having pore sizes in the range of 2 nm to 50 nm. No particular spatial organization or method of manufacture is implied by these terms. Particular mesoporous silicas have pore sizes in the range of 2 nm to 30 nm, or in the range of 2 nm to 20 nm, or in the range of 4 nm to 16 nm, or in the range of 6 nm to 14 nm.

Whenever used herein, the term "ordered" in relation to mesoporous silicas refers to silicas having at least one level of structural order, in particular having one level of structural order. In one embodiment the term "ordered" refers to ordered arrays of mesopores with regular pore size and morphology.

The terms "drug-loaded" and "loaded", when used in relation to an ordered mesoporous silica are meant to be equivalent terms.

The term "compressed" refers to a formulation that has been subjected to pressure. Various pressures can be used, for example pressures in the range of 50 to 500 MPa, or in the range of 50 to 300 MPa, or in the range of 50 to 200 MPa, or in the range of 70 to 150 MPa, or in the range of 60 to 180 MPa, or in the range of 60 to 140 MPa, or in the range of 60 to 120 MPa, or in the range of 60 to 100 MPa.

The present invention concerns compressed formulations such as tablets with drug-loaded mesoporous silica materials from which drug release remains unchanged or drug release remains within acceptable bounderies as compared to drug release from non-compressed materials. The term "within acceptable bounderies" means that drug release is sufficient to exert the desired pharmacological effect. In one embodiment this term may mean that the amount of drug released after a period of e.g. 120 minutes, or after a period of e.g. 60 minutes, or after a period of e.g. 30 minutes, shows a 30% difference, or a 20% difference, or a 10% difference, or a 5% difference, when comparing drug release from compressed materials to non-compressed materials, or when comparing drug release from materials compressed at various pressures, for example pressures within the ranges mentioned hereinafter.

As used herein in relation to percentages, "w/w" means weight/weight, "w/v" means weight/volume.

The tablet formulations of the present invention may be prepared by compressing a mixture of a mixture of an ordered mesoporous silica loaded with an active ingredient and a combination of microcrystalline cellulose and croscarmellose sodium, and optional excipients.

Various ordered mesoporous silicas may be used in the invention, in particular hexagonally ordered mesoporous silicas, which have a honeycomb structure, i.e. the two-dimensional (2D)-hexagonal (plane group, *p6mm*) mesoporous silicas. Of interest are ordered mesoporous silicas having amorphous walls of sufficient thickness as to allow adequate structural integrity. In one embodiment, the walls have a thickness that is in the range of about 2 to about 14 nm, or about 3 to about 14 nm, or about 4 to about 14 nm, or about 4 to about 10 nm, or about 4 - 8 nm, or about 4 to about 7 nm.

Of interest are ordered mesoporous silicas having a pore diameter in the range of 5-20 nm, or in the range of 4-20 nm, or in the range of 5-16 nm, or in the range of 5-14 nm, or in the range of 6-20 nm, or in the range of 6-16 nm, or in the range of 6-14 nm. The ordered mesoporous silicas do not have functionalized organic groups in the pores.

Ordered mesoporous silicas can be obtained by linking silica precursors using structure directing organic template molecules such as surfactants or block copolymers, followed by removal of the structure directing molecules. Silica precursors include silicates such as alkali silicates, e.g. sodium silicate; silicic acids; and tetraalkyl orthosilicates, e.g. tetraethyl orthosilicate (TEOS), tetramethyl orthosilicate (TMOS) and tetrapropyl orthosilicate (TPOS). Silica precursors may also be derived from polymeric forms of silica through depolymerization. Examples include pyrogenic silica, silica gel and precipitated silica. The template molecules are removed by a calcination step at increased temperatures, such as above 400°C, or above 500°C.

Ordered mesoporous silicas that can be used are the mesoporous silicas described in U.S. Patent No. 6,592,764. Amongst these are the mesopourous silica materials, which have an ordered, two-dimensional hexagonal (p6mm) honeycomb mesostructure, in particular the materials designated as SBA-15. These materials may have porous structures with high Brunauer-Emmett-Teller (BET) surface areas in the range of about 690 to about 1,040 $m^2/g$, they may have pore volumes up to about 2.5 mL/g, they may have ultra large d(100) spacings in the range of about 7.45 - 45 nm, they may have pore sizes in the range of about 4.0 - 30 nm, and they may have silica wall thicknesses in the range of about 3 - 14 nm. Such materials can be prepared using amphiphilic block copolymers, in particular triblock polyoxyalkylenes as templating agents in acidic media followed by calcination at increased temperatures.

Further ordered mesoporous silicas that can be used are the mesoporous silicas described in WO 2009/133100, in particular the silica materials referred to as COK-12. These materials, structurally similar to the SBA-15 materials in that they also have a 2-D hexagonally ordered mesostructure, have a slightly thicker silica wall, in particular a wall thickness of >4 nm, and have different silicate connectivity in the pore walls. The ratio of Q3 to Q4 silica in these materials, in particular in COK-12, as measured for example by $^{29}$Si MAS NMR, may be less than about 0.65 and preferably less than about 0.60. COK-12 materials can be obtained from the same starting materials as the SBA-15 materials, but using a modified synthesis procedure. In this procedure the silica is prepared in the presence of an amphiphilic block copolymer, in particular a triblock polyoxyalkylene, as templating agent in neutral or slightly acidic reaction conditions such as pH 5 - 8, or pH 5.5 - pH 7, or pH 6 - pH 7. Preferably a buffer is used to stabilize the pH within these ranges, for example a citrate/citric acid buffer. The triblock polyoxyalkylenes in particular are polyethylene glycol - polypropylene glycol - polyethylene glycol co-block polymers, for example the polymers sold under the trademark "Pluronic™", e.g. Pluronic 123™. For a more detailed description of the preparation of COK-12 like materials reference is made to WO 2009/133100.

In one embodiment the ordered mesoporous silica for use in the invention is SBA-15 or COK-12. Of interest are SBA-15 or COK-12 mesoporous silicas having uniform sized cylindrical pores of about 4 - 14 nm, or about 5 - 14 nm, in particular about 6 - 14 nm, or about 6 - 12 nm. Their pore volume may be in the range from about 0.8 - 1.2 mL/g, or about 0.9 - 1.1 mL/g, and their specific surface area may be in the range from about 500 - 1000 $m^2$/g, or from about 600 - 800 $m^2$/g.

The ordered mesoporous silica materials are loaded with an active ingredient, the term "loaded" meaning that the active ingredient is adsorbed at the surface of the silica, including the surface within the pores of the silica. An major part of the active ingredient is incorporated in the pores of the silica material. Such silicas with adsorbed active ingredient are referred as "loaded silicas". The terms "loaded" and "incorporated" in this context are meant to be equivalent.

The term "active ingredient" is meant to cover any pharmaceutical or other active ingredient for oral administration to humans or animals, in particular to warm-blooded animals. Pharmaceutical active ingredients comprise synthetic molecules, biomolecules, antibodies, and the like. Other active ingredients include ingredients that have an effect on the general well-being or have an effect on the outer appearance such as the skin, hair, lips, and eyes. Such ingredients include food supplements such as, for example, dietary food supplements, vitamins, minerals, fiber, fatty acids, and amino acids. Examples of such ingredients are Vitamin C, omega-3 fatty acids, carotenes, and flavonoids.

The active ingredient to be incorporated into the ordered mesoporous silica is sized such that it can fit into the cavities of the silica. In particular, one of its dimensions is smaller than the diameter of the pores, such as below about 12 nm, or below about 10nm, or below about 7 nm, or below about 5 nm, or below about 2 nm. In one embodiment, the active ingredient has a molecular weight in the range of about 200 to about 1,000 (daltons), in particular in the range of about 200 to about 800.

In one embodiment, the active ingredient when loaded in the mesoporous silica shows an increased release compared to the active ingredient as such, or to formulations containing the active ingredient and ingredients that do not influence release. Increased release may for example be an increase of 10%, or of 20%, or of 30%, or of 50%, of the weight percentage of active ingredient released under physiological conditions (pH, temperature).

In a further embodiment, the active ingredient incorporated in the mesoporous silica shows immediate release from the compressed formulations of the invention, the term "immediate release" meaning, for example, a release of at least 60% of the drug under physiological conditions (pH, temperature), such as within 60 minutes or less, such as within 30 or less, or within 20 minutes or less, or within 15 minutes or less.

In one embodiment, the active ingredient is very slightly soluble or practically insoluble in water or aqueous media, in particular physiological aqueous media. According to the manual, Pharmaceutics (M.E. Aulton), any solvent solubility is defined as the amount of a solvent (g) required to solve 1 g of a compound, whereby the following solubility qualifications are defined: 10-30 g ("soluble"); 30-100 g ("sparingly soluble"); 100-1000 g ("slightly soluble"); 1000-10000 g ("very slightly soluble" or "poorly soluble") and more than 10000 g (practically insoluble).

The pharmaceutically active ingredient may belong to the so-called BCS classes II and IV. The Biopharmaceutical Classification System (BCS) classifies drug substances based on their aqueous solubility and intestinal permeability into four classes:

Class I--High Permeability, High Solubility
Class II--High Permeability, Low Solubility
Class III--Low Permeability, High Solubility
Class IV--Low Permeability, Low Solubility

The solubility class boundary is based on the solubility of the highest dose strength in 250 ml or less of an immediate release ("IR") formulation of a drug in aqueous media over a pH range of 1.2 to 7.5 at 37°C. A drug substance has high solubility when the highest dose strength is soluble under these conditions, and has low solubility when this is not the case. Aa drug is considered highly soluble when 90% or more of an administered dose, based on a mass determination or in comparison to an intravenous reference dose, is dissolved. An immediate release drug product is considered rapidly dissolving when no less than 85% of the drug substance dissolves within 30 minutes in a volume of 900 ml or less in media of varying pH. A drug substance is considered highly permeable when the extent of absorption in humans is determined to be greater than 90% of an administered dose.

The permeability class boundary is based, directly, on measurements of the rate of mass transfer across human intestinal membrane, and, indirectly, on the extent of absorption (fraction of dose absorbed, not systemic bioavailability) of a drug substance. A drug substance is considered highly permeable when the extent of absorption in humans is greater than 90% of an administered dose. An IR (immediate release) drug substance is considered to have low permeability when the extent of absorption in humans is determined to be less than 90% of an administered dose.

BCS Class II drugs include, for example, anti-fungals, such as intraconazole, fluoconazole, terconazole, ketoconazole, griseofulvin, and griseoverdin; anti-infectives such as sulfasalazine; anti-malaria drugs (e.g. atovaquone); immune system modulators (e.g. cyclosporin); cardiovascular drugs (e.g. digoxin and spironolactone); ibuprofen (analgesic); ritonavir, nevirapine, lopinavir (antiviral); clofazinine (leprostatic); diloxanide furoate (anti-amebic); glibenclamide (anti-diabetes); nifedipine (anti-anginal); spironolactone (diuretic); sterols or steroids such as danazol; carbamazepine; anti-virals such as acyclovir; antibiotics such as amoxicillin, tetracycline, or metronidazole; acid suppressants (H2 blockers including cimetidine, ranitidine, famotidine, and nizatidine; proton pump inhibitors including omeprazole, lansoprazole, rabeprazole, esomeprazole, and pantoprozole), mucolytic agents (megaldrate).

Most BCS Class IV Drugs are lipophilic or hydrophobic drugs. Examples include acetazolamide, furosemide, tobramycin, cefuroxime, allopurinol, dapsone, doxycycline, paracetamol, nalidixic acid, clorothiazide, tobramycin, cyclosporin, tacrolimus, and paclitaxel.

Further examples of active ingredients that are poorly soluble in water include prostaglandines, e.g. prostaglandine E2, prostaglandine F2 and prostaglandine E1; cytotoxics, e.g. paclitaxel, doxorubicin, daunorubicin, epirubicin, idarubicin, zorubicin, mitoxantrone, amsacrine, vinblastine, vincristine, vindesine, dactiomycine, bleomycine; metallocenes, e.g. titanium metallocene dichloride; lipid-drug conjugates, e.g. diminazene stearate and diminazene oleate; anti-infectives such as clindamycin; antiparasitic drugs, e.g chloroquine, mefloquine, primaquine, vancomycin, vecuronium, pentamidine, metronidazole, nimorazole, tinidazole, atovaquone, buparvaquone, nifurtimoxe; anti-inflammatory drugs, e.g. methotrexate, azathioprine.

The term "active ingredient" also encompasses small antibody fragments. Examples of small antibody fragments are Fv" fragments, single-chain Fv (scFv) antibodies, antibody Fab fragments, antibody Fab' fragments, antibody fragments of heavy or light chain CDRs, or anobodies. Also encompassed are small oligonucleic acid or peptide molecules such as aptamers, for example DNA aptamers, RNA aptamers or peptide aptamers.

The ordered mesoporous silica materials can be loaded by the solvent method, the incipient wetness method, or the melt method, which methods have been described in the prior art.

In the solvent method the silica is loaded with an active ingredient by treatment with a solution of the active ingredient, particularly a solution in an organic solvent, after which the solvent is removed. The active ingredient thereby becomes adsorbed to the surface of the silica, including the surface within the pores of the silica. Appropriate organic solvents for use in this method include solvents in which the poorly water-soluble active ingredient is soluble. A solvent that in many cases is suitable to this purpose is dichloromethane. Other organic solvents that can be used comprise 1,4-dioxane, tetrahydrofuran, 2-propanol, diethyl ether, ethyl acetate, chloroform, hexafluoroisopropanol, acetone; polar aprotic solvents such as, acetonitrile, dimethylformamide N-methyl-pyrrolidinone or dimethyl sulfoxide; non-polar solvents such as hexane, benzene, toluene. For example, a solution containing about 50 mg of active ingredient per ml can be used for loading active ingredients in mesoporous silica.

In the incipient wetness method the silica is wetted with a concentrated solution of the active ingredient, while in the melt method molten active ingredient and silica are mixed. The content of the active ingredient in the mesoporous silica materials may be in the range of about 1% to about 50%, or about 10% to about 30%, o about 15% to about 25%, for example about 20%, relative to the total weight of the loaded silica material (all percentages herein being weight/weight).

In the melt method, the active ingredient is molten together with the mesoporous silica.

Microcrystalline cellulose (MCC) for use in the invention includes microcrystalline cellulose itself and mixtures with other ingredients such a silicified microcrystalline cellulose. Examples of MCCs that can be used include:

- the Avicel™ series of products available from FMC BioPolymer, in particular Avicel PH 105™, Avicel PH 101™, Avicel PH 301™;
- the microcrystalline cellulose products available from JRS Pharma, in particular Vivapur™ 105, Vivapur™ 101, Emcocel™ SP 15, Emcocel™ 50M 105, Prosolv™ SMCC 50, in particular Prosolv™ SMCC HD90;
- the microcrystalline cellulose products available from DMV under the tradename Pharmacel™, in particular Pharmacel™ 105, Pharmacel™ 101;
- the microcrystalline cellulose products available from Blanver, in particular Tabulose (Microcel)™ 101, Tabulose (Microcel)™ 103;
- the microcrystalline cellulose products available from Asahi Kasei Corporation, such as Ceolus™ PH-F20JP, Ceolus™ PH-101, Ceolus™ PH-301, Ceolus™ KG-802, Ceolus™ KG-1000.

In one embodiment, the microcrystalline cellulose is Avicel™ PH 105.

Croscarmellose sodium is an internally cross-linked sodium carboxymethyl-cellulose, which is used as a disintegrant in pharmaceutical formulations. The cross-linking reduces water solubility while still allowing the material to swell (like a sponge) and absorb many times its weight in water. As a result, it provides superior drug dissolution and disintegration characteristics.

In one embodiment, the compressed formulation comprises from 20 to 75%, or from 25 to 70%, or from 5 to 15% of ordered mesoporous silica loaded with an active ingredient. In a further embodiment, the compressed formulation comprises from 10 to 90%, or from 15 to 85%, or from 20 to 75%, or from 30 to 70%, or from 15 to 30%, or from 50 to 90%, or from 60 to 85%, of microcrystalline cellulose. In a further embodiment, the compressed formulation comprises from 3 to 20% of croscarmellose sodium, or from 5 to 20%, or from 5 to 15%, or from 7.5 to 15%, or from 10 to 15%, of croscarmellose sodium. The compressed formulation may contain from 0 to 50%, or from 0-30%, or from 0-20%, or from 5 to 15%, or from 5 to 10%, of further excipients. All percentages in this paragraph are weight/weight.

Where croscarmellose sodium is present in the formulations of this invention in low amounts, such as 3 to 10%, or 3-5%, the MCC in these formulations may be silicized MCC, used in the quantities mentioned herein. In some embodiments the MCC in the compressed formulations is silicized MCC.

The compressed formulations may also contain optional excipients. These may comprise any of the ingredients customarily employed in the art such as diluents, binders, granulating agents, glidants (flow aids), lubricants; disintegrants, sweeteners, flavors, and pigments to make the tablets visually attractive. Examples of such excipients include hydroxypropylmethyl cellulose, crospovidone, magnesium stearate, lactose, and talc.

The weight/weight ratio between MCC and Croscarmellose sodium in the compressed formulation may be in the range of 3 : 1 to 25 : 1; or in the range of 5 : 1 to 15 : 1; such as about 5 : 1; or about 10 : 1; or about 14 : 1.

The weight/weight ratio between active ingredient-loaded mesoporous silica and the MCC/Croscarmellose sodium mixture in the compressed formulation may vary but in particular is in the range of 3 : 1 to 1 : 4; or of 2 : 1 to 1 : 3; or of 1 : 1 to 1 : 3; for example about 2 : 1; or 1 : 1; or 1 : 3.

The composition of the ingredient or excipient mixtures used to prepare the compressed formulations of the invention usually will be the same as the compressed formulations obtained.

The structure and performance of ordered mesoporous silica materials as a function of pressure was determined. Release kinetics of tablets obtained by different degrees of compression showed a decrease of drug release. Without being bound by theory, it is assumed that pressure exerted during compression compromises the pore structure, e.g. by the outer pores completely collapsing and walls breaking off. Compression of ordered mesoporous silica materials, such as for example SBA-15 and COK-12, resulted in decreasing pore volume and surface area with increasing pressure. The non-loaded material had less resistance to compaction, which may be an indication that the drug molecules loaded in the pores serve as a structural support and hence, buffer the impact of the applied pressure. The overall reduction in porosity however resulted in an overall decrease in drug release.

It was further demonstrated that adding various concentrations of microcrystalline cellulose (MCC) alone to the mixture to be compressed was insufficient to overcome the negative effect of pressure on the drug release profile. Further addition of croscarmellose sodium resulted in an unaffected release behavior of the active ingredient.

As used herein, the singular includes the plural, and vice versa, the plural includes the singular. For example, the term "loaded with an active ingredient" is meant to also comprise "loaded with one or more active ingredients". The term "about" when used in relation to a numerical value has the meaning generally known in the relevant art. In certain embodiments the term "about" may be left out or it may be interpreted to mean the numerical value +10%; or $\pm 5\%$; or $\pm 2\%$; or $\pm 1\%$.

The following examples are meant to illustrate the present invention and should not be construed as limiting its scope.

Examples

Example 1: Synthesis of Mesoporous Silicas

SBA-15 was synthesized by dissolving 24 g of Pluronic P123® ethylene oxide (EO)-propylene oxide (PO) triblock copolymer ($EO_{20}PO_{70}EO_{20}$) in 240 g of 2 M HCl under stirring. Next, 50.4 g of tetraethylorthosilicate (TEOS) was diluted in 120 g of deionized $H_2O$. This TEOS mixture was then added drop-wise to the acidic Pluronic® solution under vigorous stirring at 37 °C. After 5 minutes, the mixture remained at 37 °C static synthesis conditions for 24 hours. Afterwards, the mixture temperature was then increased to 90 °C for an additional 48 hours. Subsequently, the mixture was cooled to room temperature, vacuum filtered over a 110 mm paper filter, washed with deionized water and dried. Finally, the resulting product was heated at 1°C/min to 550 °C, and calcined for 8 hours under ambient pressure to remove the Pluronic P123® from the pores of the silica material.

COK-12 was synthesized by dissolving 4.0 g of Pluronic P123® in 107.5 g deionized water under stirring followed by the addition of 3.684 g citric acid monohydrate and 2.540 g trisodium dehydrate citrate. The resulting surfactant solution was stirred for 24 hours. 10.4 g of sodium silicate solution (10% NaOH, 27% $SiO_2$, Merck, Darmstadt, Germany) was diluted with 30.0 g of water and added to the surfactant solution. The pH was measured prior to and after the sodium silicate addition. The final mixture was stirred for 5 minutes at 175 rpm with a mechanical stirrer and kept at room temperature under static synthesis conditions for 24 hours. The synthesized material was then filtered, dried at 80 °C and calcined in two steps: 8 hours at 300 °C and 8 hours at 500 °C with a 1°C/min heating rate.

He pycnometry

A Beckman model 930 gas helium pycnometer was used to measure true particle density. Samples were analyzed after immediate removal from a 40 °C vacuum oven at a reduced pressure of 1 mbar. The reported density value is the mean of three measurements.

Tapped Density

Tapped density was measured using a jolting volumeter. The 1 mm sieved samples were analyzed after immediate removal from a 105 °C oven. 35 mL were then poured into a 50 mL graduated cylinder. Samples were subjected to successive sets of 500, 750 and 1250 taps at 240 taps/min until a volume difference of <2% was achieved between sets. Reported values for bulk and tapped density are the mean of three measurements.

Particle Size Distribution Analysis

A Malvern Mastersizer™ (Hoeilaart, Belgium) was used to assess particle size distribution of the starting material with laser scattering using a HeNe laser (633 nm). All samples were suspended in water and treated with ultrasound prior to the analysis. Calculations were performed using the Mie-theory. Reported values are the average of three replicates.

Table 1 summarizes the pharmaceutically relevant powder parameters of the non-loaded OMS materials. Tapped density measurements were performed to assess compressibility and flowability of the OMS materials. The Hausner Ratio and Carr Index values were determined using Equations 1 and 2, respectively, where ρB and ρT represent bulk and tapped density, respectively.

$$H = \rho T \rho B \quad (1)$$

$$C = 1001 - \rho B \rho T \quad (2)$$

Table 1. Bulk powder properties of ordered mesoporous silica (OMS).

| OMS | ρ bulk (g/cm$^3$) | ρ tapped (g/cm$^3$) | ρ true (g/cm$^3$) | Carr Index | Hausner Ratio | PSD (μm) D (v, 0.50) |
|---|---|---|---|---|---|---|
| SBA-15 | 0.092 ± 0.006 | 0.163 ± 0.013 | 2.11 ± 0.02 | 43.8 ± 3.3 | 1.8 ± 0.1 | 13.8 ± 0.2 |
| COK-12 | 0.094 ± 0.003 | 0.144 ± 0.005 | 1.72 ± 0.04 | 34.3 ± 2.9 | 1.5 ± 0.1 | 19.0 ± 0.1 |

From a practical perspective, powders with a Carr index ≥23 and a Hausner ratio ≥1.5 are characterized as poorly flowing powders. The high values obtained indicate that both materials exhibit poor compressibility and flowability properties, a challenge for tablet development. While the 0.094 g/cm$^3$ bulk density of COK-12 is slightly higher than that of 0.092 g/cm$^3$ for SBA-15, the resulting Carr Index of 34.29 and Hausner Ratio of 1.52 are lower, indicating a better flowing material.

Example 2: Drug Loading

Itraconazole was loaded onto the silica material using the incipient wetness procedure, infusing the drug into the pores through capillary forces. A solution of 50 mg/mL of itraconazole in methylene chloride was used to load the drug into the silica. The target drug load was 20 % (wt./wt.). The damp material was then placed in a 40 °C vacuum oven at a reduced pressure of 1 mbar for a minimum of 24 hours to remove any residual methylene chloride.

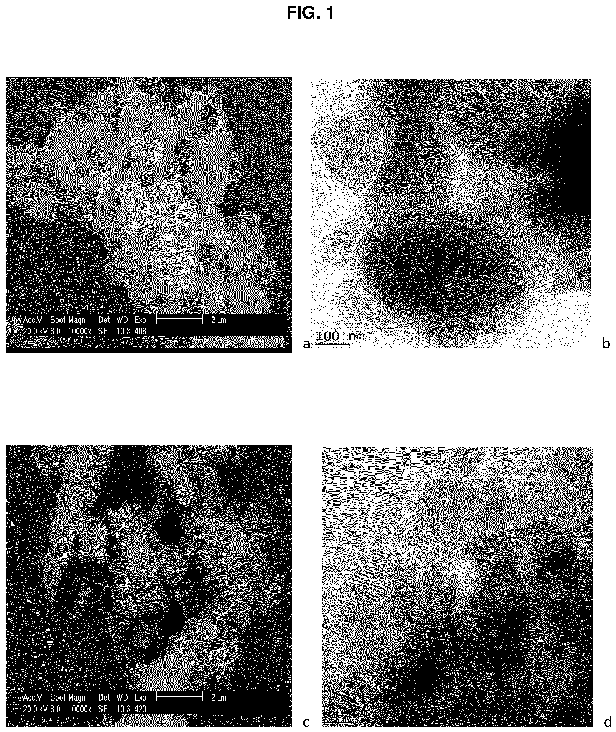

The SEM and TEM images of loaded COK-12 compressed at 0 and 480 MPa are shown in Figure 1. Comparable to SBA-15, the morphology of non-compressed COK-12 consists of smaller particles <1 μm that are covalently linked, forming larger randomly oriented conglomerates, as seen in Figure 1a. The material subjected to 480 MPa no longer exhibited these well defined separate submicron particles. However, Figure 1b reveals that the overall morphology of the larger aggregates remains intact. The TEM image in Figure 1c clearly displays the well defined hexagonal honeycomb-like pore structure of the non-compressed COK-12. Following compression to 480 MPa, small pieces of the individual particles were broken off from the surface and no longer exhibited contrast of pores, indicating heavy damage with applied pressure, as shown in Figure 1d. However, intact pores could still be observed despite the extreme pressure applied.

Example 3: Compression

For compression, mixtures of itraconazole-loaded OMS with microcrocrystalline cellulose (Avicel™) were prepared with and without croscarmellose sodium (Ac-Di-Sol™). Homogeneous samples were prepared by geometric dilutions and mixed again after pouring into a 13 mm die with a spatula immediately prior to compression. A Rodac RQPBA15 was used to manually subject the material to specific pressures of 72, 120, 240, 360, and 480 MPa for 10 seconds. The resulting sample was then ground using a mortar and pestle prior to further analysis.

$N_2$ Adsorption-desorption isotherms

Nitrogen adsorption isotherms of all silica materials were measured at -196 °C using a Micrometrics Tristar II 3020™-apparatus. Samples were pre-treated overnight at respectively, 110 °C and 250 °C for drug loaded and non-loaded silica, under a nitrogen flush. The pore volume and the surface area were calculated using the *t*-plot method of Jaroniec and Kruk (see Chem. Mater. 16:899-905, 2004). The mesopore size distribution was derived from the adsorption branches of the nitrogen isotherms using the Kruk-Jeroniec-Sayari (KJS) model.

The porosity and surface area obtained from the *t*-plot analysis of the nitrogen adsorption isotherms of both materials are listed in Table 2. The parent COK-12 and SBA-15 materials display very similar values for the pore volume and the specific surface area. Structural deterioration is caused by increasing pressure, as observed by the overall decrease in specific surface area and volume for both materials. Compared to the changes in specific surface area and volume, the KJS pore diameter size is weakly affected. While it appears that the KJS pore size diameter remains relatively unaffected, the decrease difference in pore volume and surface area support the assumption that some pores may be blocked due to partial collapse of pores and that less resistant pores completely collapse due to compression, while some pores still remain unaffected, as observed by TEM analysis. For non-loaded material, the collapse of larger pores into smaller ones resulted in an overall micropore volume increase with pressure, while the mesopore volume decreased. No microporosity for the loaded material could be detected, which is likely due to the itraconazole molecules loaded inside the pores.

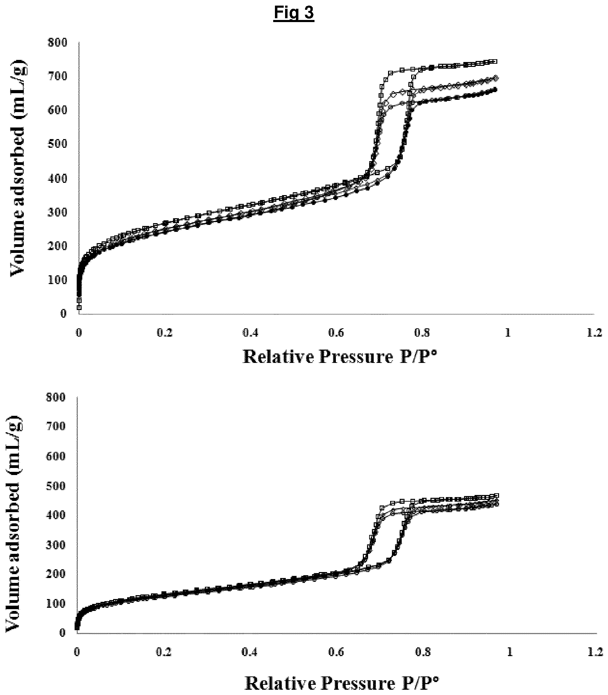

The nitrogen adsorption isotherm differences with respect to pressure of non-loaded and itraconazole-loaded COK-12 at 0, 72, and 120 MPa are illustrated in Figure 3. The isotherms are typical type IV according to the IUPAC classification for mesoporous materials. The overall decrease in volume of nitrogen adsorbed was assumed to be due to itraconazole filling the pores. Based on the larger decrease in pore volume, the non-loaded material revealed a greater sensitivity to pressure. Elongation at roughly 0.45 relative pressure in the region prior to condensation was observed in the compressed samples, indicating changes in the uniform pore structure due to partial and/or full pore collapse. In contrast, the itraconazole-loaded material maintained its overall hysteresis loop shape with only a slight decrease in volume adsorbed with increased pressure. Therefore, it may be assumed that the drug serves as a structural support during compression.

Modulated Differential Scanning Calorimetry (MDSC)

A DSC 2920 (TA Instruments, Belgium) was used to assess the physical state of itraconazole. Each loaded 4-8 mg OMS sample was heated from 30 °C to 180 °C at a 2°C/min scan rate with an amplitude of $\pm$ 0.212 °C every 40 seconds. Crystalline itraconazole was first heated from 30 °C to 180 °C at a 20°C/min scan rate. It was then quench-cooled to 30 °C and heated to 100 °C at 2°C/min using an amplitude of about 0.212 °C with a period of 40 seconds. All experiments were performed in open aluminum pans using dry nitrogen at a flow rate of 50 mL/min. Indium was used to calibrate the temperature and enthalpic response. Sapphire was used to calibrate for heat capacity. Samples were analyzed in duplicate.

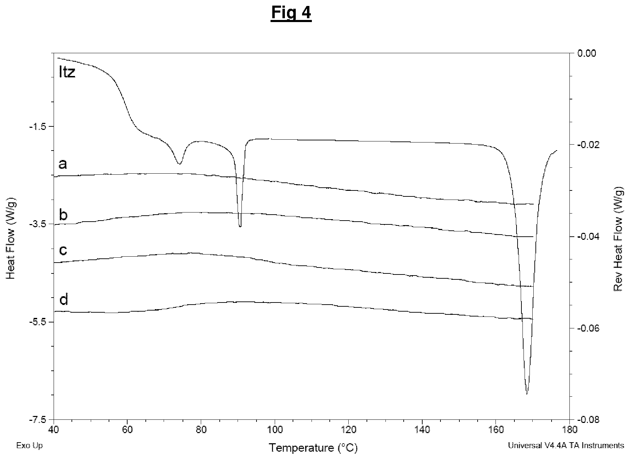

Following drug loading, the physical state of itraconazole was determined using modulated DSC. Crystalline itraconazole melts at 168 °C. Glassy itraconazole displays a glass transition at 59 °C along with two other endothermic transitions. The first is observed at 74 °C due to molecular rotational restriction and the second at 90 °C, caused by isotropic liquid transition to chiral nematic mesophase formation. Due to the state of the loaded drug, no glass transition, bulk or depressed melting temperature could be detected for either SBA-15 or COK-12, as shown in Figure 4, confirming successful loading into the pores. Furthermore, MDSC was also used to verify that the drug molecules remain in the pores following compression at 480 MPa due to a lack of crystalline peak and/or glass transition detected.

High Performance Liquid Chromatography (HPLC) Assay

Itraconazole content and dissolution were determined using HPLC system with a UV detector. UV signals were monitored at 260 nm and peaks were integrated. The mobile phase consisted of acetonitrile/0.01N tetrabutyl ammonium hydrogen sulfate (50/50, v/v), which was filtered through a 0.45 $\mu$m PTFE membrane and degassed prior to use. A Chromolith® RP-18E 100 x 4.6 mm (Merck, Darmstadt, Germany) was used at a flow rate of 1.5 mL/min and a 20 $\mu$L injection volume. For each sample, three replicates were analyzed at room temperature. The standard curves were linear over the concentration range of 0.1-300 $\mu$g/mL.

The itraconazole contents of non-compressed SBA-15 and COK-12 were 19.1 $\pm$ 0.2 (w/w.%) and 18.1 $\pm$ 0.2 (w/w.%), respectively, as measured by HPLC. Using a manual press, these samples were then subjected to pressures of 72, 120, 240, 360, and 480 MPa. Following compression, each sample was ground with a mortar and pestle prior to the release experiments.

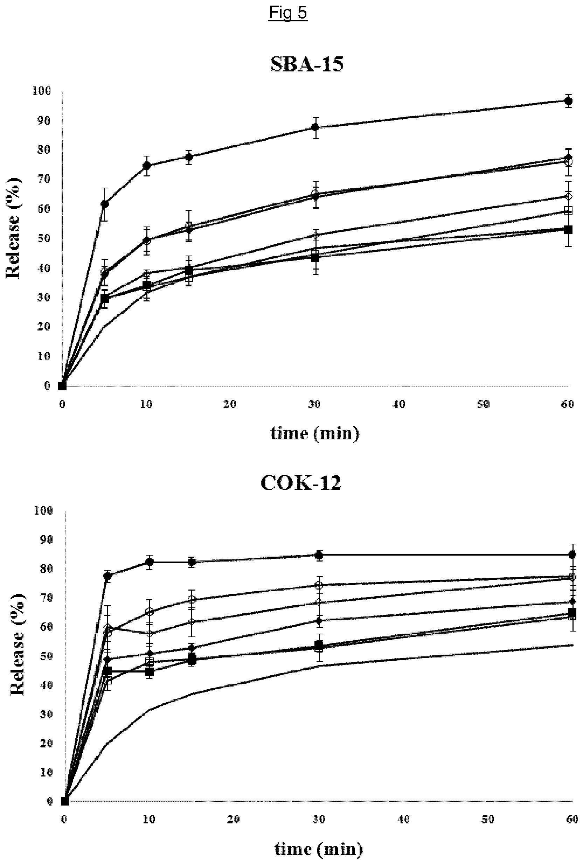

Figure 5 reveals an overall decrease in drug release with increased pressure for both materials, due to reduction in pore size and volume. Partial and/or total pore collapse would block the drug inside the pore system, therefore reducing drug release. Dissolution results are consistent with the SAXS findings in which SBA-15 displays greater sensitivity to compression than COK-12. Release with SBA-15 is faster than crystalline itraconazole up to 240 MPa, as illustrated in Figure 5. In contrast, all compressed COK-12 samples resulted in a greater percent released compared to that of crystalline itraconazole. Furthermore, non-compressed SBA-15 results in a percent release at 60 min of 75.6 $\pm$ 2.7 and reduced to 47.0 $\pm$ 4.1 after 480 MPa applied pressure. At 60 min, COK-12 released 72.0 $\pm$ 2.3 % at 0 MPa with less reduction to 63.5 $\pm$ 0.9 % after 480 MPa applied pressure, due to the larger pore sizes of COK-12 before and after compression.

Example 4: *In Vitro* Itraconazole Release

The *in vitro* drug release assessment was performed in simulated gastric fluid (SGF) + 0.5 % w/w sodium lauryl sulfate (SLS) at pH 1.2. Experiments were performed in 10 mL test tubes using a rotary mixer at 65 rpm containing 0.8 $\pm$ 0.1 mg of drug. At specific time-points (5, 10, 15, 30, and 60 minutes), samples were collected and filtered through a 0.45 $\mu$m PTFE membrane prior to HPLC analysis. All release samples were measured in triplicate.

Scanning Electron Microscopy (SEM)

Morphology was assessed using scanning electron microscopy (SEM). Images were taken with a Philips SEM XL30 FEG instrument (Philips, Eindhoven, The Netherlands) in high vacuum mode. All samples were gold-coated for imaging at room temperature.

Transmission Electron Microscopy (TEM)

TEM images were obtained using a Philips CM200 FEG (Philips, Eindhoven, The Netherlands) microscope with a field emission gun at a 200 kV operation. Each sample was prepared at room temperature by placing a small amount of the powder on a copper TEM grid which was coated with a lacey carbon film.

Small-Angle X-ray Scattering (SAXS)

SAXS patterns of powder samples placed between two pieces of tape were measured in vacuum at room temperature (25 °C) with a SAXSess mc2 instrument (Anton Paar GmbH, Austria), using line-collimated CuK$\alpha$ radiation (0.154 nm) and an image plate detector. The scattering of the tape was subtracted as background. Background subtraction and correction for instrumental broadening was performed using the SAXSquant software (Anton Paar GmbH, Austria).

From the intense reflection of the (100) diffraction measured by SAXS, the unit cell ($a = 2d_{100}/\sqrt{3}$) is determined. The KJS pore diameter (Dp) wall was calculated using Eqn. 3, where t represents the statistical film thickness, p/p° is the capillary condensation step on the adsorption isotherm, and the best-fit parameters of a, b, and c are 1.15, 0.87, and 0.27, respectively.

$$Dp = -a \log b \frac{p}{p°} + 2t + c \quad (3)$$

The wall thickness is the difference between the unit cell (a) and Dp. These results indicate that wall thickness increases with increasing compression, further supporting the assumption that some pores are completely collapsing. The greater wall thickness of the non-compressed loaded material compared to the non-loaded, is due to the itraconazole molecules depositing on the mesopore walls.

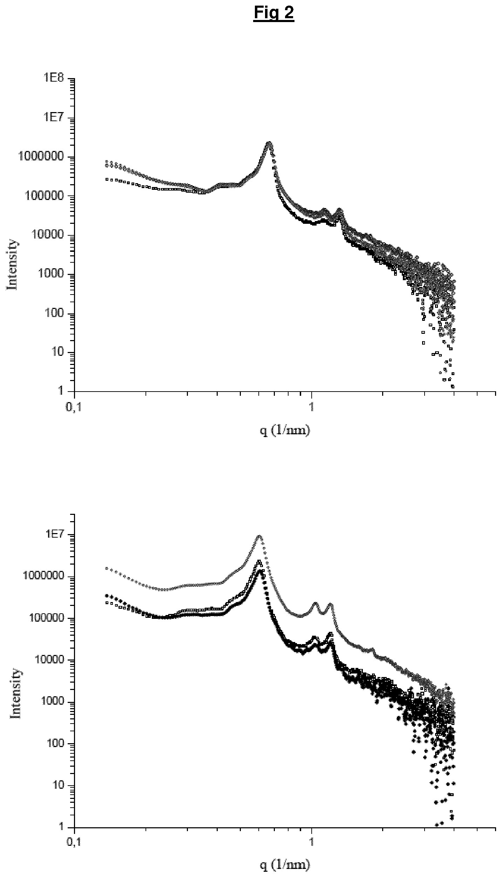

In comparing the resistance to compaction of loaded materials, Figure 2 displays SAXS results of loaded SBA-15 and COK-12 at 0, 72, and 120 MPa pressures. Neither material displays a shift in *d*-spacing. However, peak broadening was observed for SBA-15 at 72 MPa due to either reduced pore ordering, distortion or pore shape, and/or a decreased domain size. Peak broadening was not observed in loaded COK-12 until 240 MPa. The thicker wall in COK-12 and the higher condensation degree of the silicate framework determined by $^{29}$Si MAS NMR causes this material to be more robust. No significant difference between samples compressed at the same pressure was observed based on SAXS analysis.

Table 2: Textural properties of pure and drug-loaded ordered mesoporous silica materials (OMS) as determined by $N_2$ physisorption and small-angle X-ray scattering (SAXS).

| OMS | pressure (MPa) | pore diameter (nm) | wall thickness (nm) | $S_{me}$ (m$^2$/g) | $V_{me}$ (mL/g) | $V_{mi}$ (mL/g) |
|---|---|---|---|---|---|---|
| SBA-15 | 0 | 8.1 | 2.9 | 618 | 1.048 | 0.009 |
| | 72 | 8.1 | 2.9 | 562 | 0.920 | 0.002 |
| | 120 | 8.1 | 2.9 | 621 | 0.994 | 0.014 |
| | 240 | 8.1 | 2.9 | 565 | 0.840 | 0.010 |
| | 360 | 8.0 | 3.0 | 529 | 0.731 | 0.017 |
| | 480 | 7.8 | 3.3 | 487 | 0.618 | 0.020 |
| COK-12 | 0 | 9.1 | 3.0 | 652 | 1.054 | 0.013 |
| | 72 | 9.0 | 3.1 | 554 | 0.912 | 0.023 |
| | 120 | 8.9 | 3.2 | 534 | 0.851 | 0.017 |
| | 240 | 8.6 | 3.5 | 500 | 0.750 | 0.020 |
| | 360 | 8.4 | 3.7 | 498 | 0.711 | 0.027 |
| | 480 | 8.1 | 4.0 | 445 | 0.551 | 0.027 |
| Loaded SBA-15 | 0 | 7.8 | 3.3 | 360 | 0.740 | - |
| | 72 | 7.8 | 3.2 | 289 | 0.590 | - |
| | 120 | 7.7 | 3.3 | 274 | 0.546 | - |
| | 240 | 7.8 | 3.2 | 257 | 0.510 | - |
| | 360 | 7.7 | 3.3 | 231 | 0.425 | - |
| | 480 | 7.9 | 3.1 | 203 | 0.346 | - |
| Loaded COK-12 | 0 | 8.7 | 3.4 | 334 | 0.664 | - |
| | 72 | 8.7 | 3.4 | 296 | 0.594 | - |
| | 120 | 8.6 | 3.5 | 280 | 0.564 | - |
| | 240 | 8.4 | 3.7 | 256 | 0.513 | - |
| | 360 | 8.2 | 3.9 | 226 | 0.421 | - |
| | 480 | 8.0 | 4.0 | 203 | 0.362 | - |
| Mesopore surface area ($S_{me}$), Mesopore volume ($V_{me}$), Micropore volume ($V_{mi}$). | | | | | | |

Example 5: addition of MCC and croscarmellose sodium

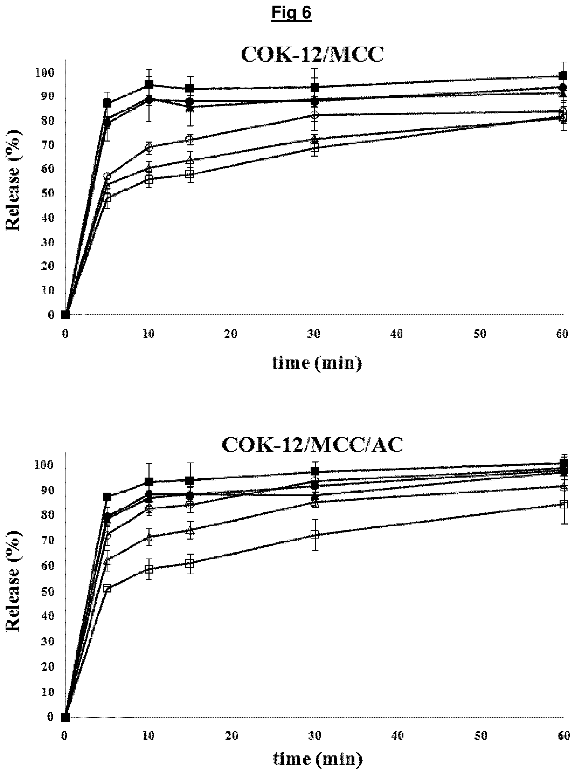

Various concentrations of MCC were added to the itraconazole-loaded OMS prior to 120 MPa compression. Samples were prepared in concentrations of drug-loaded COK-12/MCC (wt./wt.) at 7/3, 1/1, and 3/7, resulting in a drug content of 14.07 %, 9.97 %, 6.00 %, respectively. Figure 6 illustrates itraconazole release for COK-12 at each mixture before and after compression. Following 120 MPa compression, the decrease in dissolution did not fully recover with the use of MCC. However, increasing the amount of MCC did improve the release loss following compression, as seen in Figure 6. Similar findings were also observed with SBA-15.

Croscarmellose sodium (Ac-Di-Sol®) was added to the itraconazole loaded-COK-12/MCC mixture prior to compression. Samples were prepared in concentrations of itraconazole loaded-COK-12/MCC/AC (w./w.) at 66.5/28.4/5.1, 47.4/47.6/5, and 28.5/66.7/4.8, resulting in a drug content of 13.33%, 9.50%, and 5.45%, respectively. The addition of Ac-Di-Sol® improved drug release, as shown in Figure 6. The mixture of 28.5/66.7/4.8 showed no release difference before and after compression. The non-compressed sample released 79.5 ± 4.0 and 80.5 ± 3.5 at 5 and 60 minutes, respectively. Following compression at 120 MPa, release results are 72.2 ± 4.2 and 82.2 ± 1.1 at 5 and 60 minutes, respectively. Similar results were obtained for SBA-15 where no release difference following compression was achieved with the SBA-15/MCC/AC concentration of 28.5/66.5/5.0.

Example 6

In the following example COK-12 is the mesoporous silica described in example 1. The microcrystalline cellulose (MCC) used was the material available under the tradename Ceolus KG-1000™, while the croscarmellose sodium used was the material available under the tradename Ac-di-sol™.

Ezetimibe was loaded onto the ordered mesoporous silica by impregnation using the incipient wetness method. 1400 mg of ezetimibe were dissolved in 24ml of acetone. The ezetimibe solution was added to 4.00 g of COK-12 powder in 3 equal portions and after each addition the COK-12/ezetimibe mixture was homogenized with a large spatula. Upon removal of the solvent, a powder was obtained with 20% (w/w) of drug content. The ezitimibe-loaded COK-12 material is referred to as ezetimibe/COK-12.

Dissolutions were performed using a Hanson Vision® dissolution apparatus at 37°C with a paddle speed of 50 RPM. The *in vitro* drug release assessment was performed in simulated gastric fluid (SGF) + 0.5 % w/v sodium lauryl sulfate (SLS) at pH 1.2. Experiments were performed in 10 mL test tubes using a rotary mixer at 65 rpm containing 0.8 ± 0.1 mg of drug. At specific time-points (5, 10, 15, 30, and 60 minutes), samples of 1.0 mL were taken and filtered through a 0.45 μm PTFE membrane prior to HPLC analysis. The volume withdrawn was replaced by the same amount of fresh medium. All release samples were measured in duplicate.

The tablets were compared in dissolution experiments together with the ezetimibe/COK-12 powder formulation. The dissolution medium used was 0,01M pH 7 phosphate buffer + 0,1% SLS prepared as follows: 2.68 g $Na_2HPO_4.7H_2O$ + 1g SLS were dissolved in 900 mL Milli-Q water and diluted to 1.01 with Milli-Q water. 1.56g $NaH_2PO_4.2H_2O$ + 1g SLS were dissolved in 900ml Milli-Q water and diluted to 1.01 with Milli-Q water. Both solutions were mixed together to obtain a dissolution medium with pH 7.

Milli-Q™ is trademark for 'ultrapure' water of "Type 1", as defined by various authories (e.g. ISO 3696), as well as their devices for producing such water. The preparation of Milli-Q™ involves purification processes with successive steps of filtration and deionisation, to achieve a purity expediently characterized in terms of resistivity (typically 18.2 MΩ·cm).

Table 3 shows the tablet composition of the two formulations that were used for making tablets with a dose of 10 mg. In this table "%" refers to the weight/weight (w/w) of the ingredient to the total weight of the formulation.

Table 3: Tablet blends with Ezetimibe/COK-12

| Ingredient | Tablet formulation 1 | | Tablet formulation 2 | |
|---|---|---|---|---|
| | % | mg | % | mg |
| Ezetimibe/COK-12 | 21.96 | 54.9 | 21.96 | 54.9 |
| Lactose | 50.28 | 125.70 | 54.03 | 135.07 |
| MCC | 16.76 | 41.90 | 18.01 | 45.03 |
| Croscarmellose sodium | 10.00 | 25.00 | 5.00 | 12.50 |
| Mg stearate | 1.00 | 2.50 | 1.00 | 2.50 |
| Total | 100 | 250 | 100 | 250 |

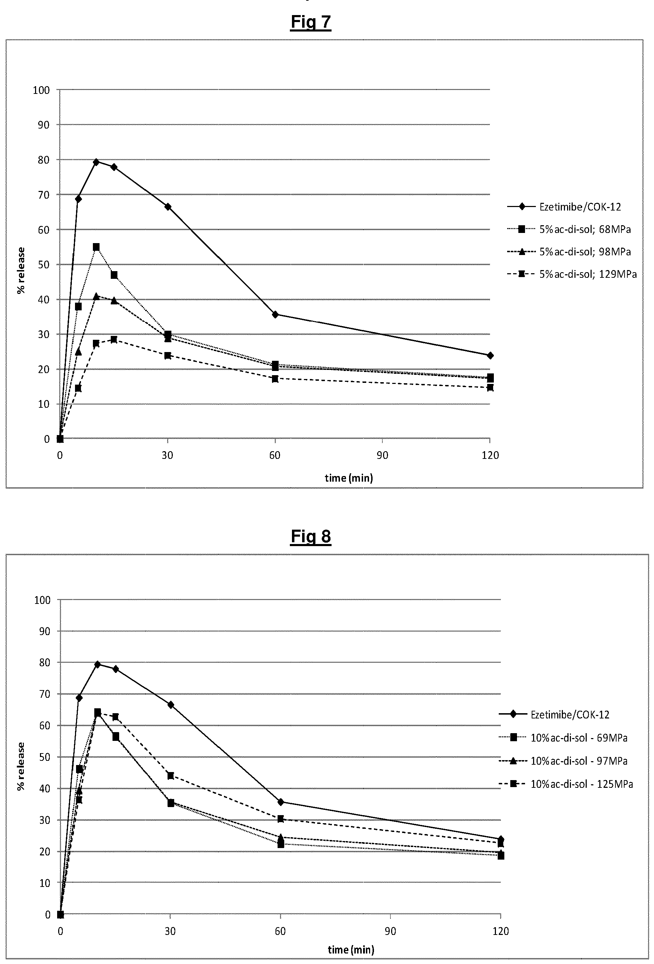

The tablets prepared from Tablet formulation 1 were tested in dissolution experiments next to the ezetimibe/COK-12 powder formulation. The results are shown in Figure 7, which depicts the release from these tablets, which contained 5% croscarmellose sodium, in 900 mL 0.01M pH 7 phosphate buffer + 0.1% SLS at 37°C and a paddle speed of 50 RPM. Ezetimibe release was satisfactory although release decreased to some extend with increased compression pressure.

The tablets with 10% croscarmellose sodium were tested in similar dissolution experiments next to the ezetimibe/COK-12 powder formulation. The results are shown in Figure 8, which depicts the release from 20% w/w Ezetimibe/COK-12 tablets in 900 mL 0.01M pH 7 phosphate buffer + 0.1% SLS at 37°C and a paddle speed of 50 RPM. Ezetimibe release approached that of the ezetimibe/COK-12 powder formulation. Release was not influenced to a relevant extend with increased compression pressures.

Figure 8 shows the dissolution/release of 20% w/w ezetimibe/COK-12 tablets with 10% croscarmellose sodium in 900 mL 0.01 M pH 7 phosphate buffer + 0.1% SLS at 37°C and a paddle speed of 50 RPM, compressed using various pressures.

Example 7

Nebivolol was loaded onto COK-12 mesoporous silica using the procedure described in example 6. The thus obtained formulation is referred to as nebivolol/COK-12.
Two tablets compositions were evaluated (see Table 4). Tablet 1 did not contain croscarmelose sodium, while Tablet 2 contained 8% w/w croscarmelose sodium.

Table 4: Tablet compositions

|  | Tablet formulation 1 | Tablet formulation 2 |
| --- | --- | --- |
| Ingredient | % (w/w) | % (w/w) |
| Nebivolol/COK-12 | 13.6 | 13.6 |
| MCC | 84.4 | 80.4 |
| Croscarmellose sodium | - | 4.00 |
| Mg stearate | 1.00 | 1.00 |
| Talc | 1.00 | 1.00 |
| Total | 100 | 100 |

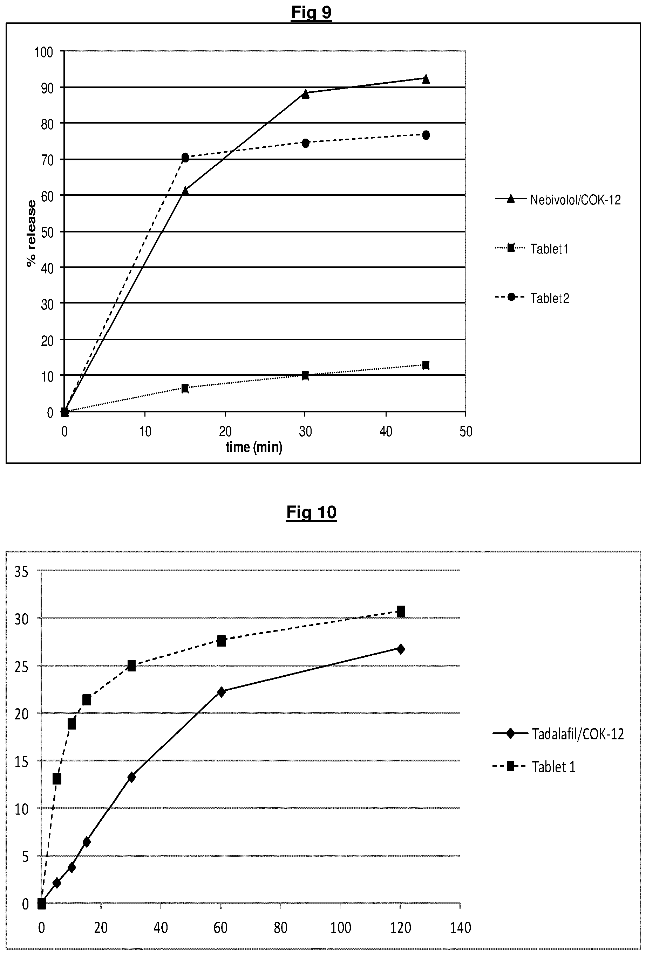

In both cases such amounts of the mixture of excipients were used to obtain a tablet weight of 50 mg. The results of the release experiments are shown in Figure 9.

Example 8: Release of tadalafil

The composition of the excipient blends used to prepare Tadalafil/CMO tablets is shown in Table 5.

Table 5: Composition of the tablet blends of the Tadalafil/COK-12 tablet

| Excipient | %(w/w) |
| --- | --- |
| Tadalafil | 31.18 |
| PanExcea™ MHC300G | 57.82 |
| Croscarmellose sodium | 10 |
| Mg stearate | 1 |

PanExcea™ MHC300G is a tradename of a mixture of 89% (w/w) of MCC, 2% (w/w) of hydroxypropylmethyl cellulose, and 9% (w/w) of crospovidone.
Such amounts of the mixture of excipients were used to obtain a tablet tablet weight of 152 mg. The results of the release experiments are shown in Figure 10.

**Claims**

1. A compressed formulation comprising an ordered mesoporous silica loaded with an active ingredient, a combination of micro crystalline cellulose (MCC) and croscarmellose sodium, and optional further excipients, wherein said ordered mesoporous silica has been subjected to a calcination step.

2. The formulation of claim 1, wherein the pore size of the ordered mesoporous silica is in the range of 5 to 30 nm.

3. The formulation of claim 1, wherein the pore size of the ordered mesoporous silica is in the range of 6 to 14 nm.

4. The formulation according to any one of claims 1 to 3, in the form of a tablet.

5. The formulation according to any of claims 1 to 4, wherein the ordered mesoporous silica is a hexagonally-ordered two-dimensional array of uniform sized cylindrical pores.

6. The formulation according to any of claims 1 to 5, wherein the ordered mesoporous silica has a silica wall thickness that is in the range of 3 - 14 nm.

7. The formulation according to any of claims 1 to 6, wherein the active ingredient is very slightly soluble or poorly soluble.

8. The formulation according to any of claims 1 to 7, wherein the weight/weight ratio between MCC and Croscarmellose sodium in the formulation is in the range of 3 : 1 to 25 : 1.

9. The formulation according to any of claims 1 to 8, wherein the weight/weight ratio between active ingredient-loaded mesoporous silica and the MCC/Croscarmellose sodium mixture in the formulation is in the range of 3 : 1 to 1 : 4.

10. The formulation according to any of claims 1 to 9, wherein the formulation comprises from 20 to 75% (w/w) of ordered mesoporous silica loaded with an active ingredient.

11. The formulation according to any of claims 1 to 10, wherein the formulation comprises-from 15 to 85% (w/w) of microcrystalline cellulose.

12. The formulation according to any of claims 1 to 11, wherein the formulation comprises from 3 to 15% (w/w) of croscarmellose sodium.

13. The formulation according to any of claims 1 to 12, wherein the formulation comprises-from 0 to 50% (w/w) of further excipients.

14. A process for preparing a compressed formulation as specified in any of claims 1 - 13, wherein the process comprises compressing a mixture of an ordered mesoporous silica loaded with an active ingredient, a combination of microcrystalline cellulose and croscarmellose sodium, and optional excipients.

**Patentansprüche**

1. Komprimierte Formulierung, umfassend ein geordnetes mesoporöses Siliciumdioxid, das mit einem Wirkstoff beladen ist, eine Kombination von mikrokristalliner Cellulose (MCC) und Croscarmellose-Natrium und fakultative weitere Hilfsstoffe, wobei das geordnete mesoporöse Siliciumdioxid einem Calcinierungsschritt unterworfen wurde.

2. Formulierung nach Anspruch 1, wobei die Porengröße des geordneten mesoporösen Siliciumdioxids im Bereich von 5 bis 30 nm liegt.

3. Formulierung nach Anspruch 1, wobei die Porengröße des geordneten mesoporösen Siliciumdioxids im Bereich von 6 bis 14 nm liegt.

4. Formulierung nach einem der Ansprüche 1 bis 3 in Form einer Tablette.

5. Formulierung nach einem der Ansprüche 1 bis 4, wobei es sich bei dem geordneten mesoporösen Siliciumdioxid um eine hexagonal geordnete zweidimensionale Anordnung von einheitlich großen zylindrischen Poren handelt.

6. Formulierung nach einem der Ansprüche 1 bis 5, wobei das geordnete poröse Siliciumdioxid eine Siliciumdioxid-Wanddicke aufweist, die im Bereich von 3-14 nm liegt.

7. Formulierung nach einem der Ansprüche 1 bis 6, wobei der Wirkstoff sehr schwer löslich oder schlecht löslich ist.

8. Formulierung nach einem der Ansprüche 1 bis 7, wobei das Gewicht/Gewicht-Verhältnis zwischen MCC Croscarmellose-Natrium der Formulierung im Bereich von 3:1 bis 25:1 liegt.

9. Formulierung nach einem der Ansprüche 1 bis 8, wobei das Gewicht/Gewicht-Verhältnis zwischen wirkstoffbeladenem mesoporösem Siliciumdioxid und der MCC/Croscarmellose-Natrium-Mischung in der Formulierung im Bereich von 3:1 bis 1:4 liegt.

10. Formulierung nach einem der Ansprüche 1 bis 9, wobei die Formulierung 20 bis 75 % (w/w) geordnetes mesoporöses Siliciumdioxid, das mit einem Wirkstoff beladen ist, umfasst.

11. Formulierung nach einem der Ansprüche 1 bis 10, wobei die Formulierung 15 bis 85 % (w/w) mikrokristalline Cellulose umfasst.

12. Formulierung nach einem der Ansprüche 1 bis 11, wobei die Formulierung 3 bis 15 % (w/w) Croscarmellose-Natrium umfasst.

13. Formulierung nach einem der Ansprüche 1 bis 12, wobei die Formulierung 0 bis 50 % (w/w) weitere Hilfsstoffe umfasst.

14. Verfahren zur Herstellung einer komprimierten Formulierung gemäß einem der Ansprüche 1-13, bei dem man eine Mischung aus einem geordneten mesoporösen Siliciumdioxid, das mit einem Wirkstoff beladen ist, einer Kombination von mikrokristalliner Cellulose und Croscarmellose-Natrium und fakultativen weiteren Hilfsstoffen komprimiert.

**Revendications**

1. Formulation comprimée comprenant une silice mésoporeuse ordonnée chargée avec un principe actif, une combinaison de cellulose microcristalline (MCC) et de croscarmellose sodique, et des excipients supplémentaires éventuels, dans laquelle ladite silice mésoporeuse ordonnée a été soumise à une étape de calcination.

2. Formulation selon la revendication 1, dans laquelle la taille de pore de la silice mésoporeuse ordonnée est comprise dans la plage de 5 à 30 nm.

3. Formulation selon la revendication 1, dans laquelle la taille de pore de la silice mésoporeuse ordonnée est comprise dans la plage de 6 à 14 nm.

4. Formulation selon l'une quelconque des revendications 1 à 3, sous la forme d'un comprimé.

5. Formulation selon l'une quelconque des revendications 1 à 4, dans laquelle la silice mésoporeuse ordonnée est un réseau à deux dimensions ordonné de manière hexagonale de pores cylindriques de taille uniforme.

6. Formulation selon l'une quelconque des revendications 1 à 5, dans laquelle la silice mésoporeuse ordonnée a une épaisseur de paroi de silice qui est comprise dans la plage de 3 à 14 nm.

7. Formulation selon l'une quelconque des revendications 1 à 6, dans laquelle le principe actif est très légèrement soluble ou peu soluble.

8. Formulation selon l'une quelconque des revendications 1 à 7, dans laquelle le rapport poids/poids entre la MCC et la croscarmellose sodique dans la formulation est dans la plage de 3:1 à 25:1.

9. Formulation selon l'une quelconque des revendications 1 à 8, dans laquelle le rapport poids/poids entre la silice mésoporeuse chargée avec le principe actif et le mélange de MCC/croscarmellose sodique dans la formulation est dans la plage de 3:1 à 1:4.

10. Formulation selon l'une quelconque des revendications 1 à 9, dans laquelle la formulation comprend de 20 à 75 % (p/p) de silice mésoporeuse ordonnée chargée avec un principe actif.

11. Formulation selon l'une quelconque des revendications 1 à 10, dans laquelle la formulation comprend de 15 à 85 % (p/p) de cellulose microcristalline.

12. Formulation selon l'une quelconque des revendications 1 à 11, dans laquelle la formulation comprend de 3 à 15 % (p/p) de croscarmellose sodique.

13. Formulation selon l'une quelconque des revendications 1 à 12, dans laquelle la formulation comprend de 0 à 50 % (p/p) d'excipients supplémentaires.

14. Procédé de préparation d'une formulation comprimée telle que spécifiée selon l'une quelconque des revendications 1 à 13, dans lequel le procédé comprend la compression d'un mélange d'une silice mésoporeuse ordonnée chargée avec un principe actif, d'une combinaison de cellulose microcristalline (MCC) et de croscarmellose sodique, et d'excipients éventuels.

**FIG. 1**

**Fig 2**

## Fig 3

**Fig 4**

Fig 5

SBA-15

COK-12

Fig 6

COK-12/MCC

COK-12/MCC/AC

## Fig 7

## Fig 8

## Fig 9

## Fig 10

**EP 2 646 005 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060018966 A
- US 6592764 B
- WO 2009133100 A

### Non-patent literature cited in the description

- **MELLAERTS et al.** *Eur J Pharm Biopharm,* 2008, vol. 69, 223-230
- **GHEDINI et al.** *Micropor and Mesopor Mater,* 2010, vol. 132, 258-267
- **JARONIEC ; KRUK.** *Chem. Mater.,* 2004, vol. 16, 899-905